# EUROPEAN PATENT APPLICATION

(11) **EP 4 787 135 A1**
(43) Date of publication of application: **05.08.2026**
(21) Application number: 24871443.8
(22) Date of filing: 08.07.2024
(51) Int. Cl.: G06F 3/04845, G06F 3/0482, G06F 3/04815, G06T 19/00

(54) **IMAGE DISPLAY PROCESSING DEVICE, IMAGE DISPLAY PROCESSING METHOD, AND PROGRAM**

(30) Priority: 27.09.2023 JP 2023166321
(71) Applicant: FUJIFILM Corporation, Tokyo 106-8620 (JP)
(72) Inventor: NODA, Rena, Tokyo 106-8620 (JP)
(74) Representative: Dehns Germany Partnerschaft mbB
(86) International application number: PCT/JP2024/024666
(87) International publication number: WO 2025/069639

(57) **Abstract**

An image display processing device includes at least one processor. The processor is configured to receive designation of a position of a three-dimensional image including a plurality of objects on a display screen, determine a target object that is a target of an operation related to display from among the plurality of objects according to a preset priority based on the designated position, or present, as a candidate for the target object, at least one of a plurality of objects that overlap each other in a depth direction at the designated position or a plurality of objects that include the designated position in a region of the object, and receive an input for an operation related to display for the target object.

## Description

### BACKGROUND OF THE INVENTION

### 1. Field of the Invention

The disclosed technology relates to an image display processing device, an image display processing method, and a program.

### 2. Description of the Related Art

The following technologies are known as technologies related to image display processing. For example, JP2020-000863A discloses a medical image processing system including a display device that displays an image, an input device that receives an input from an operator, a data storage unit that stores at least one data set capable of creating a three-dimensional image representing a three-dimensional arrangement of a plurality of anatomical structures, and an image display control unit that controls display of the image on the display device in accordance with the input received by the input device.

JP2014-90807A discloses a medical image diagnostic apparatus comprising a first designation unit that designates a position in a three-dimensional image that is a rendering result of volume data including a first region and a second region that covers at least a part of the first region in accordance with an input by an operation of an input unit, a setting unit that variably sets a transparency of the second region in accordance with a movement of the position designated by the first designation unit, and a first display control unit that performs rendering of the volume data based on the transparency set by the setting unit and performs control of displaying a three-dimensional image obtained by the rendering on a display unit.

### SUMMARY OF THE INVENTION

In a system that handles a three-dimensional image including a plurality of three-dimensional objects, an operation related to display is performed on a specific object selected from among the plurality of objects as a target. The "operation related to display" is, for example, switching between display and non-display, setting a transparency, rotation, movement, and addition of information (annotation, texture) for an operation target object.

In particular, in a medical three-dimensional image such as a computed tomography (CT) image, a plurality of objects corresponding to respective structures of interest such as a blood vessel, an organ, and a tumor are arranged in a complicated manner. In such a three-dimensional image, selecting an operation target object (hereinafter, referred to as a target object) from among the plurality of objects is complicated.

For example, the selection and operation of the target object are performed in the following procedure. In the following description, a case where a transparency is set for a specific object included in the three-dimensional image will be described as an example. The transparency is set using a user interface (UI) in a list format that lists identification names or identification colors of all the objects included in the three-dimensional image. The UI is displayed in a region different from a display region of the three-dimensional image on a display screen. The user selects an identification name or an identification color corresponding to an object that is a target of the transparency setting from among the plurality of identification names or identification colors listed in the list. Thereafter, the user sets the transparency using a UI for performing an operation of a slider bar, numerical input, or the like on the selected object.

According to the above-described aspect, in order to select the target object from among the plurality of objects included in the three-dimensional image, the user needs to move the gaze from the target object to the UI or move the mouse, which hinders the user's thinking. In addition, the user needs to recognize a correspondence relationship between the plurality of objects included in the three-dimensional image and the identification name or the identification color displayed on the list before selecting and operating on the target object, and in a case where the correspondence relationship is erroneously recognized, an operation error occurs.

The disclosed technology has been made in view of the above points, and an object thereof is to reduce a burden on the user in a case of selecting the target object that is a target of an operation related to display from among the plurality of objects included in the three-dimensional image.

An image processing device according to the disclosed technology is an image display processing device comprising at least one processor, in which the processor is configured to receive designation of a position of a three-dimensional image including a plurality of objects on a display screen, determine a target object that is a target of an operation related to display from among the plurality of objects according to a preset priority based on the designated position, or present, as a candidate for the target object, at least one of a plurality of objects that overlap each other in a depth direction at the designated position or a plurality of objects that include the designated position in a region of the object, and receive an input for an operation related to display for the target object.

The processor may be configured to present the candidate for the target object using a list that lists identification names of the plurality of objects that are the candidates for the target object. The processor may be configured to display the list in the vicinity of the designated position. The processor may be configured to present the identification names listed in the list to be arranged in an order corresponding to an arrangement order in the depth direction of the plurality of objects that overlap each other in the depth direction.

The processor may be configured to determine, as the target object, or present, as the candidate for the target object, an object that is selected according to the preset priority from among the plurality of objects for at least one of the plurality of objects that overlap each other in the depth direction at the designated position or the plurality of objects that include the designated position in the region of the object. The processor may be configured to determine the priority for at least one of the plurality of objects that overlap each other in the depth direction at the designated position or the plurality of objects that include the designated position in the region of the object based on a transparency set for each of the plurality of objects.

The processor may be configured to present all the objects included in the three-dimensional image as the candidate for the target object in a case where there is no object displayed at the designated position. The processor may be configured to present all the objects set not to be displayed from among the objects included in the three-dimensional image as the candidate for the target object in a case where there is no object displayed at the designated position.

The operation related to the display of the target object may include at least one of switching between display and non-display, setting a transparency, rotation, movement, or addition of information for the target object. The processor may be configured to receive, as an input for the operation related to the display of the target object, an input by a mouse operation or a touch operation after the target object is determined. The processor may be configured to display a user interface for the operation related to the display of the determined target object in the vicinity of the target object.

An image display processing method according to the disclosed technology is an image display processing method in which at least one processor included in an image display processing device executes a process of receiving designation of a position of a three-dimensional image including a plurality of objects on a display screen, determining a target object that is a target of an operation related to display from among the plurality of objects according to a preset priority based on the designated position, or presenting, as a candidate for the target object, at least one of a plurality of objects that overlap each other in a depth direction at the designated position or a plurality of objects that include the designated position in a region of the object, and receiving an input for an operation related to display for the target object.

A program according to the disclosed technology is a program causing at least one processor included in an image display processing device to execute a process of receiving designation of a position of a three-dimensional image including a plurality of objects on a display screen, determining a target object that is a target of an operation related to display from among the plurality of objects according to a preset priority based on the designated position, or presenting, as a candidate for the target object, at least one of a plurality of objects that overlap each other in a depth direction at the designated position or a plurality of objects that include the designated position in a region of the object, and receiving an input for an operation related to display for the target object.

According to the disclosed technology, it is possible to reduce a burden on the user in a case of selecting the target object that is a target of an operation related to display from among the plurality of objects included in the three-dimensional image.

### BRIEF DESCRIPTION OF THE DRAWINGS

FIG. 1 is a diagram showing an example of a configuration of an image management system according to an embodiment of the disclosed technology.
FIG. 2 is a diagram showing an example of a hardware configuration of an image display processing device according to the embodiment of the disclosed technology.
FIG. 3 is a functional block diagram showing an example of a functional configuration of an image display processing device according to the embodiment of the disclosed technology.
FIG. 4 is a diagram showing an example of a display form of a three-dimensional image according to the embodiment of the disclosed technology.
FIG. 5 is a diagram showing an example of a display form of the three-dimensional image according to the embodiment of the disclosed technology.
FIG. 6 is a diagram showing an example of a display form of the three-dimensional image according to the embodiment of the disclosed technology.
FIG. 7 is a diagram showing an example of a display form of the three-dimensional image according to the embodiment of the disclosed technology.
FIG. 8 is a diagram showing an example of a display form of the three-dimensional image according to the embodiment of the disclosed technology.
FIG. 9 is a diagram showing an example of a display form of the three-dimensional image according to the embodiment of the disclosed technology.
FIG. 10 is a flowchart showing an example of a flow of a process executed in the image display processing device according to the embodiment of the disclosed technology.
FIG. 11 is a diagram showing an example of a display form of the three-dimensional image according to the embodiment of the disclosed technology.
FIG. 12 is a diagram showing an example of a display form of the three-dimensional image according to the embodiment of the disclosed technology.
FIG. 13 is a diagram showing an example of a display form of a three-dimensional image according to a comparative example.

### DESCRIPTION OF THE PREFERRED EMBODIMENTS

Hereinafter, an example of an embodiment of the disclosed technology will be described with reference to the drawings. The same or equivalent components and parts in the respective drawings are denoted by the same reference numerals, and the duplicated description will be omitted.

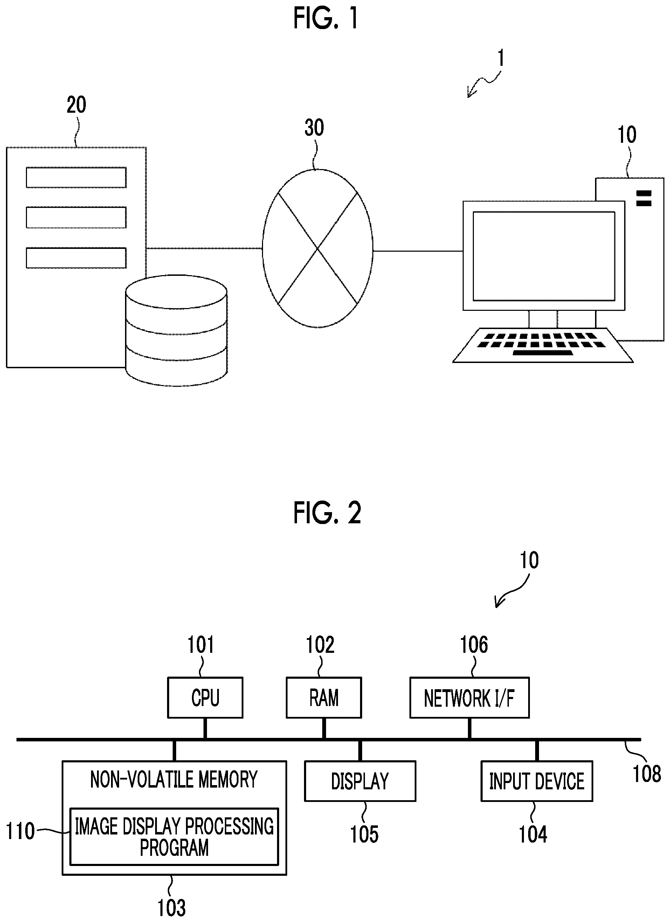

FIG. 1 is a diagram showing an example of a configuration of an image management system 1 according to the embodiment of the disclosed technology. The image management system 1 includes a server 20 and an image display processing device 10. The image management system 1 may constitute, for example, a picture archiving and communication system (PACS), and the server 20 may be, for example, an image server that stores and manages medical images such as an X-ray image, a CT image, and a magnetic resonance imaging (MRI) image. The image display processing device 10 is a viewer terminal that is connected to the server 20 via a network 30 and that can read out and display an image stored in the server 20. The image display processing device 10 performs a process related to display of a three-dimensional image including a plurality of three-dimensional objects.

FIG. 2 is a diagram showing an example of a hardware configuration of the image display processing device 10. The image display processing device 10 includes a central processing unit (CPU) 101, a random-access memory (RAM) 102, a non-volatile memory 103, an input device 104 including a keyboard and a mouse, a display 105, and a network interface 106. These pieces of hardware are connected to a bus 108.

The display 105 may be a touch panel display. The network interface 106 is an interface for connecting the image display processing device 10 to the network 30.

The non-volatile memory 103 is a non-volatile storage medium such as a hard disk and a flash memory. An image display processing program 110 is stored in the non-volatile memory 103. The RAM 102 is a work memory for the CPU 101 to execute a process. The CPU 101 loads the image display processing program 110 into the RAM 102, and executes the process according to the image display processing program 110. The CPU 101 is an example of a "processor" in the disclosed technology.

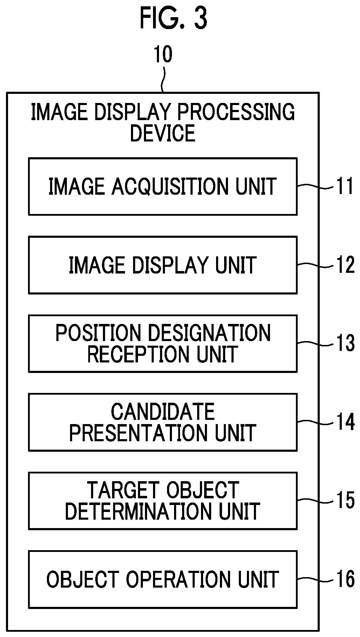

FIG. 3 is a functional block diagram showing an example of a functional configuration of the image display processing device 10. The image display processing device 10 includes an image acquisition unit 11, an image display unit 12, a position designation reception unit 13, a candidate presentation unit 14, a target object determination unit 15, and an object operation unit 16. The CPU 101 executes the image display processing program 110, whereby the image display processing device 10 functions as the image acquisition unit 11, the image display unit 12, the position designation reception unit 13, the candidate presentation unit 14, the target object determination unit 15, and the object operation unit 16.

The image acquisition unit 11 acquires a three-dimensional image that is a processing target of the image display processing device 10 based on an instruction from the user performed via the input device 104. The image acquisition unit 11 may acquire the three-dimensional image that is the processing target by downloading the three-dimensional image from the server 20. In addition, in a case where the image that is the processing target is stored in the non-volatile memory 103, the image that is the processing target may be read out and acquired from the non-volatile memory 103.

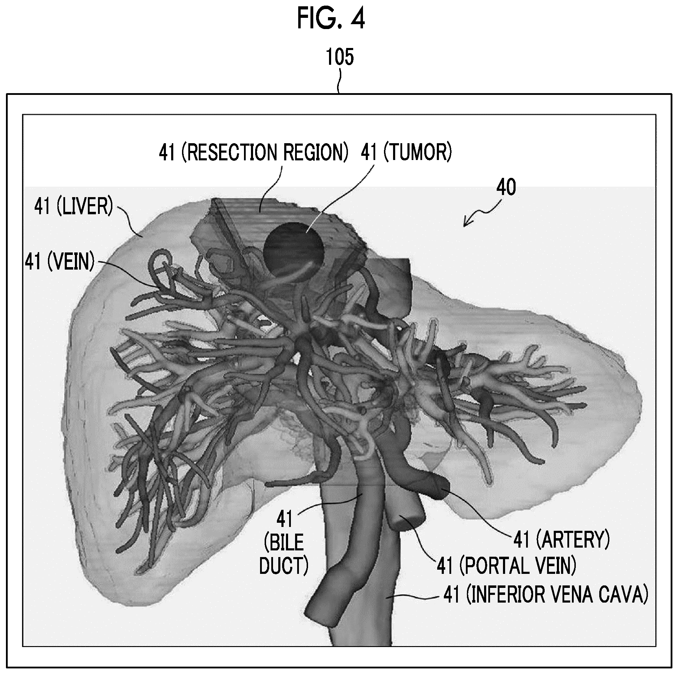

The image display unit 12 displays the three-dimensional image acquired by the image acquisition unit 11 on the display 105. FIG. 4 is a diagram showing an example of a three-dimensional image 40 displayed on the display 105. FIG. 4 shows the three-dimensional image 40 around a liver generated based on a CT image. The three-dimensional image 40 includes a plurality of three-dimensionally displayed objects 41 corresponding to a liver, a tumor, a resection region, an artery, a vein, a portal vein, an inferior vena cava, and a bile duct, respectively. In the three-dimensional image 40, information indicating which anatomical type each of the plurality of objects 41 corresponds to is associated in advance. This association can be realized, for example, by using a known segmentation technique. The image display unit 12 may display the plurality of objects 41 in different colors.

In the image display processing device 10, an operation related to the display can be performed individually on the plurality of objects 41. The "operation related to display" is, for example, switching between display and non-display, setting a transparency, rotation, movement, and addition of information (annotation, texture) for a target object. The user can designate an object that is a target of an operation related to the display (hereinafter, referred to as a target object). The designation of the target object can be performed, for example, by indicating a display position of the target object on a screen on which the three-dimensional image 40 is displayed by a mouse pointer (mouse cursor). In addition, in a case where the display 105 is a touch panel display, the target object may be designated by a touch operation. The position designation reception unit 13 receives the designation of the position on the display screen of the three-dimensional image 40 by the user operation.

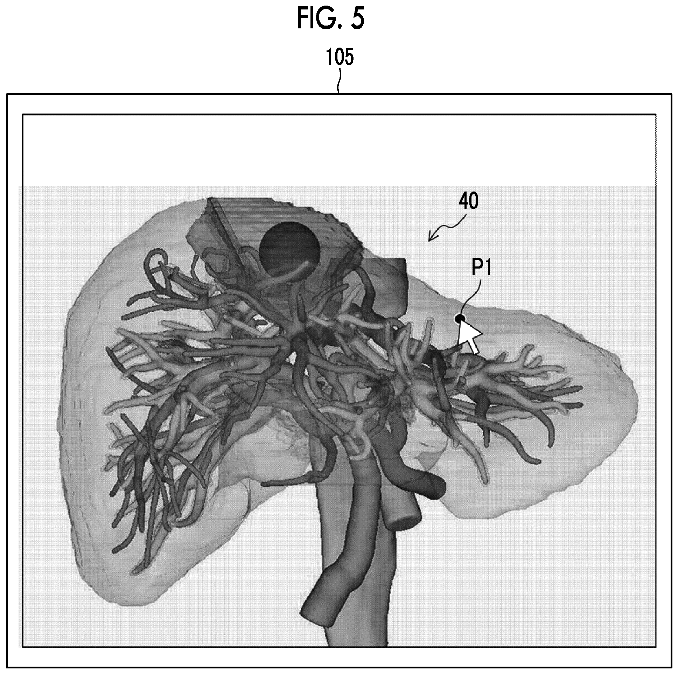

The target object determination unit 15 determines the corresponding object based on the position designation received by the position designation reception unit 13. In a case where only one object is present at the designated position, the target object determination unit 15 determines the one object present at the designated position as the target object. For example, in a case where a position P1 shown in FIG. 5 is designated, the target object determination unit 15 determines an object corresponding to "liver" as the target object based on the designation of the position P1.

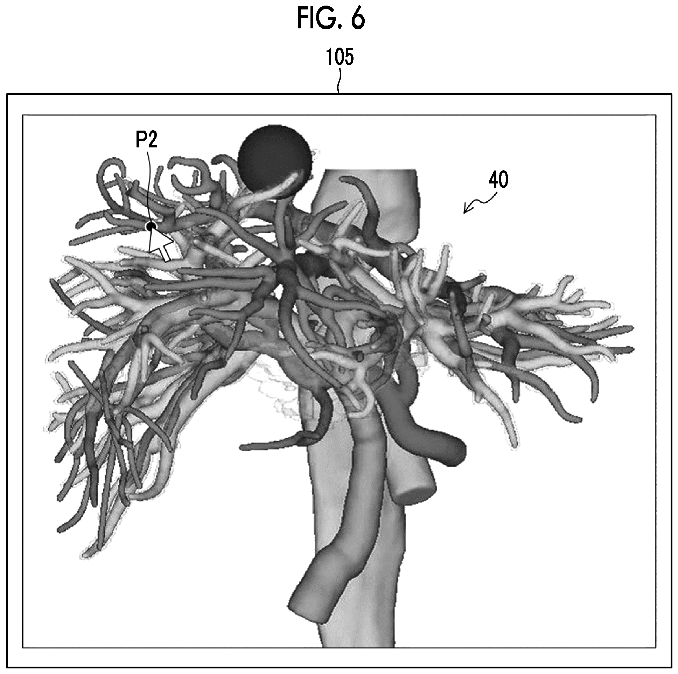

FIG. 6 shows the three-dimensional image 40 in which the object corresponding to "liver" is not displayed. In a case where a position P2 shown in FIG. 6 is designated, the target object determination unit 15 determines an object corresponding to "vein" as the target object based on the designation of the position P2.

On the other hand, in a case where a plurality of objects overlap each other in the depth direction at the designated position, the target object determination unit 15 determines the target object through the presentation of the candidate for the target object by the candidate presentation unit 14 described below and the selection designation of the user.

In a case where a plurality of objects overlap each other in the depth direction at a position related to the designation received by the position designation reception unit 13, the candidate presentation unit 14 presents the plurality of objects that overlap each other in the depth direction at the designated position as the candidate for the target object.

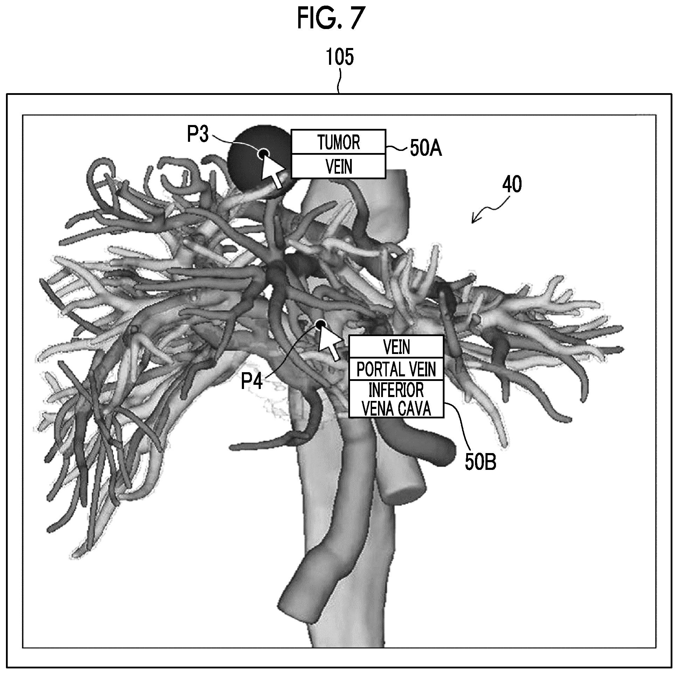

For example, in a case where "tumor" and "vein" overlap each other at a designated position P3 shown in FIG. 7, the candidate presentation unit 14 sets each of the objects corresponding to the "tumor" and the "vein" as the candidate for the target object based on the designation of the position P3. The candidate presentation unit 14 presents the candidate for the target object using a list 50A that lists the identification names (the "tumor" and the "vein") of each of the objects corresponding to the "tumor" and the "vein" set as the candidate for the target object. The list 50A is displayed in the vicinity of the designated position P3.

In addition, for example, in a case where "vein", "portal vein", and "inferior vena cava" overlap each other at a designated position P4 shown in FIG. 7, the candidate presentation unit 14 sets each of the objects corresponding to the "vein", the "portal vein", and the "inferior aorta" as the candidate for the target object based on the designation of the position P4. The candidate presentation unit 14 presents the candidate for the target object using a list 50B that lists the identification names (the "vein", the "portal vein", and the "inferior aorta") of each of the objects corresponding to the "vein", the "portal vein", and the "inferior aorta" set as the candidate for the target object. The list 50B is displayed in the vicinity of the designated position P4.

The candidate presentation unit 14 may present the identification names of the objects listed in the list to be arranged in an order corresponding to an arrangement order in the depth direction of the plurality of objects that overlap each other in the depth direction. For example, a list in which the identification names of the objects are arranged in order from the top may be presented in order from the object present closest to the front side toward the back side.

In addition, the candidate presentation unit 14 presents a plurality of objects that include the position related to the designation received by the position designation reception unit 13 in a region of the object as the candidate for the target object. That is, in a case where the designated position is included in the region of the first object and is also included in the region of the second object, the first and second objects are presented as the candidates for the target object, respectively.

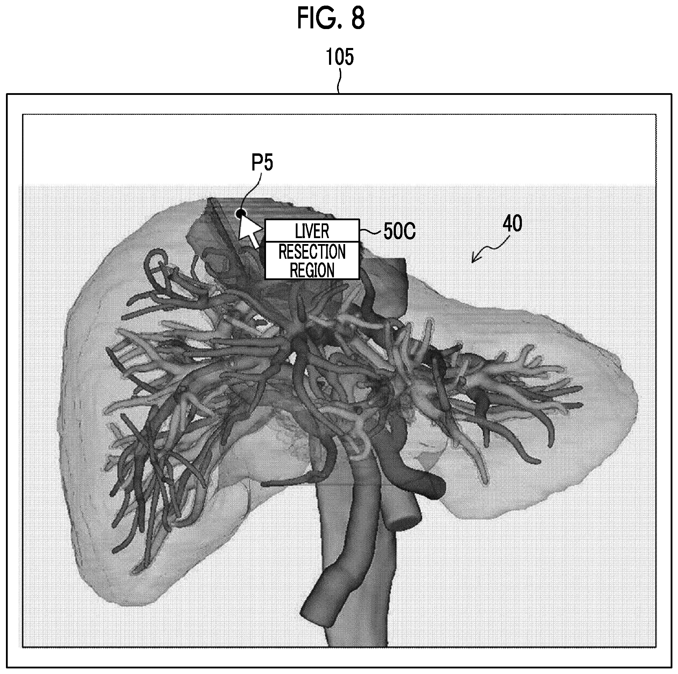

For example, each of the objects corresponding to "liver" and "resection region" that include the designated position P5 shown in FIG. 8 in the region is set as the candidate for the target object. The candidate presentation unit 14 presents the candidate for the target object using a list 50C that lists the identification names (the "liver" and the "resection region") of each of the objects corresponding to the "liver" and the "resection region" set as the candidate for the target object. The list 50C is displayed in the vicinity of the designated position P5.

The user can select an object to be the target object from among the plurality of objects presented as the candidates for the target object. The selection of the object can be performed using the input device 104. For example, the selection of the object may be performed by performing a predetermined mouse operation in a state where the mouse pointer is disposed on the list. More specifically, the plurality of objects listed in the list may be toggled by performing a right click in a state where the mouse pointer is disposed on the list, and then performing a long click, a mouse wheel operation, or a shortcut key operation. The target object determination unit 15 determines the target object based on the selection designation of the object to be the target object.

The object operation unit 16 receives an input for an operation related to the display for the target object determined by the target object determination unit 15. After the target object is determined, the user can instruct the operation related to the display for the target object by, for example, a mouse operation or a touch operation on the screen. The object operation unit 16 receives, as an input for the operation related to the display of the target object, an input by the mouse operation or the touch operation on the screen after the target object is determined. For example, in a case where a drag operation using the mouse is performed after the target object is determined, the object operation unit 16 may change the transparency of the target object according to an operation amount and reflect the change in the display of the three-dimensional image 40.

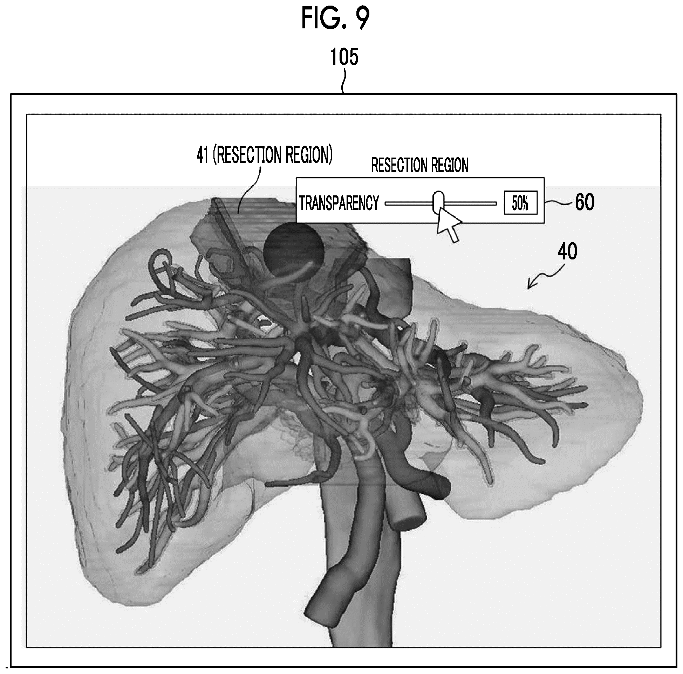

The object operation unit 16 may display an operation UI for the operation related to the display for the determined target object in the vicinity of the target object. FIG. 9 shows an example in which an operation UI 60 for setting the transparency for the object 41 corresponding to the "resection region" set as the target object is displayed in the vicinity of the object 41 corresponding to the "resection region". The user can set the transparency of the object 41 corresponding to the "resection region" by operating the operation UI 60. The object operation unit 16 sets the transparency of the object 41 corresponding to the "resection region" to a value designated by the operation of the operation UI 60 and reflects the setting in the display of the three-dimensional image 40.

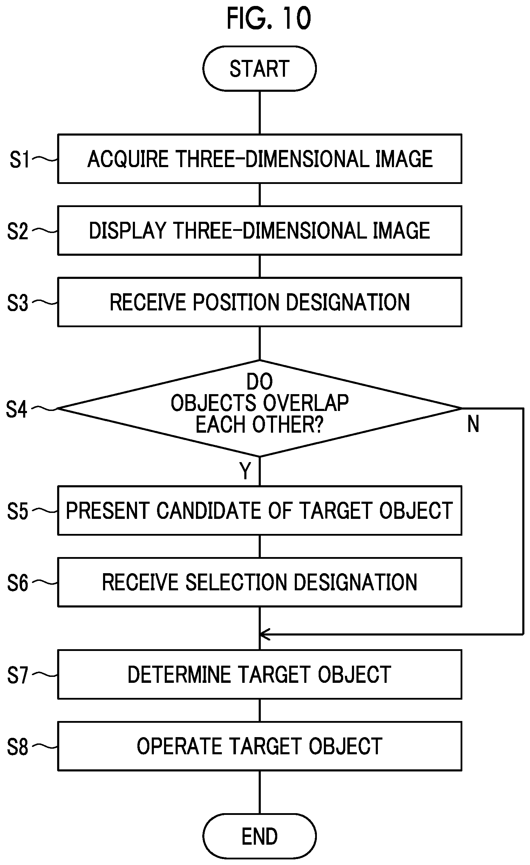

FIG. 10 is a flowchart showing an example of a flow of a process executed by the CPU 101 executing the image display processing program 110.

In step S1, the image acquisition unit 11 acquires the three-dimensional image that is the processing target of the image display processing device 10 based on the instruction from the user performed via the input device 104. In step S2, the image display unit 12 displays the three-dimensional image acquired in step S1 on the display 105. In step S3, the position designation reception unit 13 receives the designation of the position on the display screen of the three-dimensional image by the user operation.

In step S4, the CPU 101 determines whether or not a plurality of objects overlap each other in the depth direction at the position related to the designation received in step S3. In a case where it is determined that a plurality of objects overlap each other in the depth direction at the designated position, the process proceeds to step S5, and in a case where it is determined that a plurality of objects do not overlap each other in the depth direction at the designated position, the process proceeds to step S7.

In step S5, the candidate presentation unit 14 sets the plurality of objects that overlap each other in the depth direction at the designated position as the candidate for the target object. In addition, the candidate presentation unit 14 sets a plurality of objects that include the designated position in the region of the object as the candidate for the target object. The candidate presentation unit 14 displays the list that lists the identification names of each of the plurality of objects set as the candidate for the target object in the vicinity of the designated position.

In step S6, the target object determination unit 15 receives the selection designation of the object to be the target object from among the plurality of objects presented as the candidates for the target object.

In a case where it is determined in the determination in step S4 that a plurality of objects do not overlap each other in the depth direction, in step S7, the target object determination unit 15 determines the one object present at the designated position as the target object. On the other hand, in a case where the selection designation of the object is received in step S6, in step S7, the target object determination unit 15 determines the object related to the selection designation from among the plurality of objects presented as the candidates for the target object in step S5 as the target object.

In step S8, the object operation unit 16 receives the input for the operation related to the display for the object determined as the target object in step S7. The object operation unit 16 performs the operation related to the display of the target object (switching between display and non-display, setting a transparency, rotation, movement, and addition of information) based on the received input.

Hereinafter, a modification example of the image display processing device 10 will be described. A priority may be set for each of the plurality of objects included in the three-dimensional image in the selection of the target object and the presentation of the candidate. In this case, the target object determination unit 15 may determine, as the target object, an object that is selected according to a preset priority from among the plurality of objects for at least one of the plurality of objects that overlap each other in the depth direction at the designated position or the plurality of objects that include the designated position in the region of the object.

For example, in a case where "vein", "portal vein", and "inferior vena cava" overlap each other at the designated position P4 shown in FIG. 7 and the highest priority is set for the object corresponding to the "portal vein" among these sites, the target object determination unit 15 may determine the object corresponding to the "portal vein" as the target object based on the designation of the position P4.

In addition, for example, in a case where a relatively high priority is set for the object corresponding to the "resection region" among the objects corresponding to the "liver" and the "resection region" that include the designated position P5 shown in FIG. 8 in the region, the target object determination unit 15 may determine the object corresponding to the "resection region" as the target object based on the designation of the position P5.

In addition, the candidate presentation unit 14 may present, as the candidate for the target object, an object that is selected according to a preset priority from among the plurality of objects for at least one of the plurality of objects that overlap each other in the depth direction at the designated position or the plurality of objects that include the designated position in the region of the object.

For example, in a case where "vein", "portal vein", and "inferior vena cava" overlap each other at the designated position P4 shown in FIG. 7 and the priorities of the objects corresponding to the "vein" and the "portal vein" are relatively high and the priority of the object corresponding to the "inferior vena cava" is relatively low among these sites, the candidate presentation unit 14 may present the objects corresponding to the "vein" and the "portal vein" as the candidate for the target object based on the designation of the position P4.

The priority may be optionally set by the user based on an anatomical importance or the like. In addition, the priority may be determined based on the transparency set for each of the plurality of objects for at least one of the plurality of objects that overlap each other in the depth direction at the designated position or the plurality of objects that include the designated position in the region of the object. The transparency can be set for each of the plurality of objects included in the three-dimensional image. The transparency is a degree of transparency in a display color of the object. As the transparency is higher, the transparency of the object is higher, and the visibility of the object behind is higher. The image display processing device 10 may set a higher priority for the object as the transparency set for the object is lower. For example, in a case where "vein", "portal vein", and "inferior vena cava" overlap each other at the designated position P4 shown in FIG. 7 and the transparency of the object corresponding to the "portal vein" is the lowest among these, the target object determination unit 15 may determine the object corresponding to the "portal vein" as the target object based on the designation of the position P4.

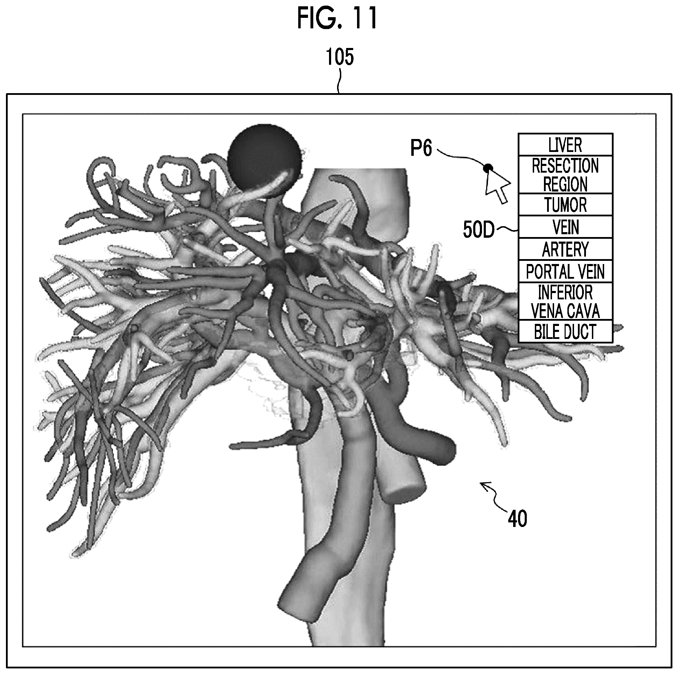

In addition, in a case where there is no object displayed at the designated position, the candidate presentation unit 14 may present all the objects included in the three-dimensional image as the candidates for the target object. The "case where there is no object displayed at the designated position" includes both a case where there is no object at the designated position and a case where the object present at the designated position is set not to be displayed. For example, as shown in FIG. 11, the candidate presentation unit 14 may present the candidate for the target object using a list 50D that lists the identification names "liver", "resection region", "tumor", "vein", "artery", "portal vein", "inferior vena cava", and "bile duct" of all the objects included in the three-dimensional image 40 based on the designation of the position P6 where there is no displayed object. The list 50D is displayed in the vicinity of the designated position P6.

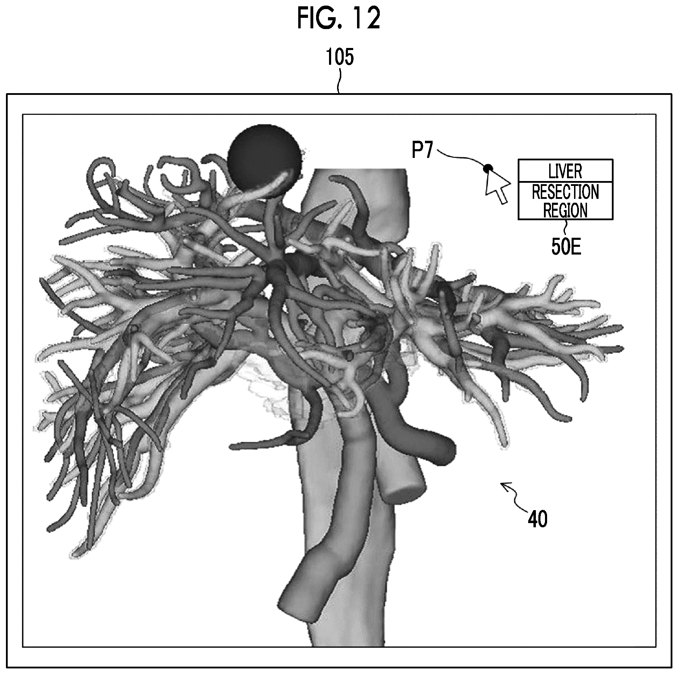

In addition, in a case where there is no object displayed at the designated position, the candidate presentation unit 14 may present all the objects set not to be displayed from among the objects included in the three-dimensional image as the candidate for the target object. For example, in a case where the objects corresponding to the "liver" and the "resection region" are set not to be displayed, as shown in FIG. 12, the candidate presentation unit 14 may present the candidate for the target object using a list 50E that lists the identification names "liver" and "resection region" of all the objects set not to be displayed based on the designation of the position P7 where there is no displayed object. The list 50E is displayed in the vicinity of the designated position P7.

As described above, the image display processing device 10 according to the embodiment of the disclosed technology receives the designation of the position of the three-dimensional image including the plurality of objects on the display screen, determines the target object that is the target of the operation related to the display from among the plurality of objects according to the preset priority based on the designated position, or presents, as the candidate for the target object, at least one of the plurality of objects that overlap each other in the depth direction at the designated position or the plurality of objects that include the designated position in the region of the object, and receives the input for the operation related to the display for the target object.

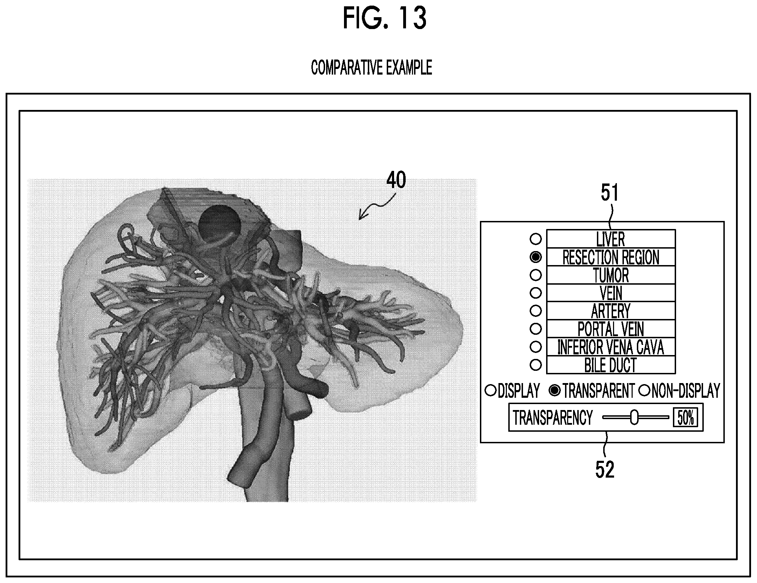

Here, an example of a procedure of the selection and the operation of the target object in the system according to the comparative example will be described with reference to FIG. 13. In the following description, a case where a transparency is set for a specific object included in the three-dimensional image will be described as an example. The transparency is set using a UI 51 in a list format that lists the identification names or the identification colors of all the objects included in the three-dimensional image 40. The UI 51 is displayed in a region different from a display region of the three-dimensional image 40 on the display screen. The user selects an identification name corresponding to an object that is a target of the transparency setting from among the plurality of identification names listed in the list of the UI 51. Thereafter, the user sets the transparency using a UI 52 for performing an operation of a slider bar, numerical input, or the like on the selected object.

According to the aspect of the comparative example, in order to select the target object from among the plurality of objects included in the three-dimensional image 40, the user needs to move the gaze from the target object to the UI 51 or move the mouse, which hinders the user's thinking. In addition, the user needs to recognize a correspondence relationship between the plurality of objects included in the three-dimensional image 40 and the identification name displayed on the list of the UI 51 before selecting and operating on the target object, and in a case where the correspondence relationship is erroneously recognized, an operation error occurs.

On the other hand, according to the image display processing device 10 according to the embodiment of the disclosed technology, since the target object is automatically determined or the candidate for the target object is presented, the movement of the gaze and the movement of the mouse of the user accompanying the selection of the target object can be minimized, and the burden on the user in a case of selecting the target object can be reduced. As a result, since the user's thinking is not hindered, the workability can be improved. In addition, according to the image display processing device 10, in the selection and the operation of the target object, since it is not necessary for the user to recognize the correspondence relationship between the plurality of objects included in the three-dimensional image and the identification names or the identification colors thereof, or the burden thereof can be minimized, the occurrence of the operation error due to the erroneous recognition of the correspondence relationship can be suppressed.

In addition, according to the image display processing device 10, since at least one of the plurality of objects that overlap each other in the depth direction at the designated position or the plurality of objects that include the designated position in the region of the object is presented as the candidate for the target object, the candidate for the target object can be limited while including an appropriate object in the candidate for the target object.

In addition, according to the image display processing device 10, the candidate for the target object is presented using a list that lists the identification names of the plurality of objects that are the candidates for the target object. The list is displayed in the vicinity of the designated position. In addition, the identification names listed in the list are presented in a state of being arranged in an order corresponding to the arrangement order in the depth direction of the plurality of objects that overlap each other in the depth direction. As a result, it is easy to understand the candidate for the target object and to select the target object from the candidate.

In addition, according to the image display processing device 10 according to the present embodiment, the input by the mouse operation or the touch operation after the target object is determined is received as the input for the operation related to the display of the target object. Alternatively, a user interface for the operation related to the display of the determined target object is displayed in the vicinity of the target object. As a result, it is possible to perform the operation related to the display of the corresponding object without the movement of the gaze and the movement of the mouse.

In addition, in the system according to the comparative example shown in FIG. 13, it is necessary to secure a display region dedicated to the UIs 51 and 52, and the display region of the three-dimensional image 40 is narrowed. On the other hand, according to the image display processing device 10 according to the present embodiment, since the UI for the selection and the operation of the target object is displayed in a pop-up manner at the designated position, it is not necessary to secure the display region dedicated to the UI. As a result, since the display region of the three-dimensional image 40 can be increased as compared with the system according to the comparative example, the visibility and the workability are improved.

In the above description, a case where the disclosed technology is applied to the medical three-dimensional image has been described as an example, but the disclosed technology is not limited to this aspect. The disclosed technology can be applied to the three-dimensional image handled in computer-aided design (CAD) used in various fields such as games, industrial products, and architecture. In addition, in the above description, a case where the image display processing device 10 executes each process in the flowchart shown in FIG. 10 has been described as an example, but a part or all of these processes may be executed by the server 20.

As hardware for executing processes in each functional unit of the image display processing device 10, various processors as shown below can be used. The processor may be a CPU that executes software (programs) and functions as various processing units. Furthermore, the processor may be a programmable logic device (PLD) such as an FPGA whose circuit configuration is changeable. Moreover, the processor may have a circuit configuration that is specially designed to execute a specific process, such as an application-specific integrated circuit (ASIC).

Each functional unit of the image display processing device 10 may be configured by one of the various processors described above, or may be configured by a combination of the same or different kinds of two or more processors (for example, a combination of a plurality of FPGAs or a combination of the CPU and the FPGA). In addition, a plurality of functional units may be configured by one processor.

In the above embodiment, the aspect has been described in which the image display processing program 110 is stored (installed) in the non-volatile memory 103 in advance, but the disclosed technology is not limited thereto. The image display processing program 110 may be provided in a form recorded in a recording medium such as a compact disc read-only memory (CD-ROM), a digital versatile disc read-only memory (DVD-ROM), and a universal serial bus (USB) memory. In addition, the image display processing program 110 may be downloaded from an external device via a network.

In regard to the embodiment described above, the following supplementary notes will be further disclosed.

### (Supplementary Note 1)

An image display processing device comprising:
at least one processor,
in which the processor is configured to:
   receive designation of a position of a three-dimensional image including a plurality of objects on a display screen;
   determine a target object that is a target of an operation related to display from among the plurality of objects according to a preset priority based on the designated position, or present, as a candidate for the target object, at least one of a plurality of objects that overlap each other in a depth direction at the designated position or a plurality of objects that include the designated position in a region of the object; and
   receive an input for an operation related to display for the target object.

### (Supplementary Note 2)

The image display processing device according to Supplementary Note 1,
in which the processor is configured to present the candidate for the target object using a list that lists identification names of the plurality of objects that are the candidates for the target object.

### (Supplementary Note 3)

The image display processing device according to Supplementary Note 2,
in which the processor is configured to display the list in the vicinity of the designated position.

### (Supplementary Note 4)

The image display processing device according to Supplementary Note 2 or 3,
in which the processor is configured to present the identification names listed in the list to be arranged in an order corresponding to an arrangement order in the depth direction of the plurality of objects that overlap each other in the depth direction.

### (Supplementary Note 5)

The image display processing device according to any one of Supplementary Notes 1 to 4,
in which the processor is configured to determine, as the target object, or present, as the candidate for the target object, an object that is selected according to the preset priority from among the plurality of objects for at least one of the plurality of objects that overlap each other in the depth direction at the designated position or the plurality of objects that include the designated position in the region of the object.

### (Supplementary Note 6)

The image display processing device according to Supplementary Note 5,
in which the processor is configured to determine the priority for at least one of the plurality of objects that overlap each other in the depth direction at the designated position or the plurality of objects that include the designated position in the region of the object based on a transparency set for each of the plurality of objects.

### (Supplementary Note 7)

The image display processing device according to any one of Supplementary Notes 1 to 6,
in which the processor is configured to present all the objects included in the three-dimensional image as the candidate for the target object in a case where there is no object displayed at the designated position.

### (Supplementary Note 8)

The image display processing device according to any one of Supplementary Notes 1 to 7,
in which the processor is configured to present all the objects set not to be displayed from among the objects included in the three-dimensional image as the candidate for the target object in a case where there is no object displayed at the designated position.

### (Supplementary Note 9)

The image display processing device according to any one of Supplementary Notes 1 to 8,
in which the operation related to the display of the target object includes at least one of switching between display and non-display, setting a transparency, rotation, movement, or addition of information for the target object.

### (Supplementary Note 10)

The image display processing device according to any one of Supplementary Notes 1 to 9,
in which the processor is configured to receive, as an input for the operation related to the display of the target object, an input by a mouse operation or a touch operation after the target object is determined.

### (Supplementary Note 11)

The image display processing device according to Supplementary Note 1,
in which the processor is configured to display a user interface for the operation related to the display of the determined target object in the vicinity of the target object.

### (Supplementary Note 12)

An image display processing method in which at least one processor included in an image display processing device executes a process of:
receiving designation of a position of a three-dimensional image including a plurality of objects on a display screen;
determining a target object that is a target of an operation related to display from among the plurality of objects according to a preset priority based on the designated position, or presenting, as a candidate for the target object, at least one of a plurality of objects that overlap each other in a depth direction at the designated position or a plurality of objects that include the designated position in a region of the object; and
receiving an input for an operation related to display for the target object.

### (Supplementary Note 13)

A program causing at least one processor included in an image display processing device to execute a process of:
receiving designation of a position of a three-dimensional image including a plurality of objects on a display screen;
determining a target object that is a target of an operation related to display from among the plurality of objects according to a preset priority based on the designated position, or presenting, as a candidate for the target object, at least one of a plurality of objects that overlap each other in a depth direction at the designated position or a plurality of objects that include the designated position in a region of the object; and
receiving an input for an operation related to display for the target object.

The disclosure of Japanese Patent Application No. 2023-166321 filed on September 27, 2023 is incorporated in the present specification by reference. In addition, all documents, patent applications, and technical standards described in the present specification are incorporated herein by reference to the same extent as in a case of being specifically and individually noted that individual documents, patent applications, and technical standards are incorporated in the present specification by reference.

## Claims

1. An image display processing device comprising:
at least one processor,
wherein the processor is configured to:
receive designation of a position of a three-dimensional image including a plurality of objects on a display screen;
determine a target object that is a target of an operation related to display from among the plurality of objects according to a preset priority based on the designated position, or present, as a candidate for the target object, at least one of a plurality of objects that overlap each other in a depth direction at the designated position or a plurality of objects that include the designated position in a region of the object; and
receive an input for an operation related to display for the target object.

2. The image display processing device according to claim 1,
wherein the processor is configured to present the candidate for the target object using a list that lists identification names of the plurality of objects that are the candidates for the target object.

3. The image display processing device according to claim 2,
wherein the processor is configured to display the list in the vicinity of the designated position.

4. The image display processing device according to claim 2,
wherein the processor is configured to present the identification names listed in the list to be arranged in an order corresponding to an arrangement order in the depth direction of the plurality of objects that overlap each other in the depth direction.

5. The image display processing device according to claim 1,
wherein the processor is configured to determine, as the target object, or present, as the candidate for the target object, an object that is selected according to the preset priority from among the plurality of objects for at least one of the plurality of objects that overlap each other in the depth direction at the designated position or the plurality of objects that include the designated position in the region of the object.

6. The image display processing device according to claim 5,
wherein the processor is configured to determine the priority for at least one of the plurality of objects that overlap each other in the depth direction at the designated position or the plurality of objects that include the designated position in the region of the object based on a transparency set for each of the plurality of objects.

7. The image display processing device according to claim 1,
wherein the processor is configured to present all the objects included in the three-dimensional image as the candidate for the target object in a case where there is no object displayed at the designated position.

8. The image display processing device according to claim 1,
wherein the processor is configured to present all the objects set not to be displayed from among the objects included in the three-dimensional image as the candidate for the target object in a case where there is no object displayed at the designated position.

9. The image display processing device according to claim 1,
wherein the operation related to the display of the target object includes at least one of switching between display and non-display, setting a transparency, rotation, movement, or addition of information for the target object.

10. The image display processing device according to claim 1,
wherein the processor is configured to receive, as an input for the operation related to the display of the target object, an input by a mouse operation or a touch operation after the target object is determined.

11. The image display processing device according to claim 1,
wherein the processor is configured to display a user interface for the operation related to the display of the determined target object in the vicinity of the target object.

12. An image display processing method in which at least one processor included in an image display processing device executes a process of:
receiving designation of a position of a three-dimensional image including a plurality of objects on a display screen;
determining a target object that is a target of an operation related to display from among the plurality of objects according to a preset priority based on the designated position, or presenting, as a candidate for the target object, at least one of a plurality of objects that overlap each other in a depth direction at the designated position or a plurality of objects that include the designated position in a region of the object; and
receiving an input for an operation related to display for the target object.

13. A program causing at least one processor included in an image display processing device to execute a process of:
receiving designation of a position of a three-dimensional image including a plurality of objects on a display screen;
determining a target object that is a target of an operation related to display from among the plurality of objects according to a preset priority based on the designated position, or presenting, as a candidate for the target object, at least one of a plurality of objects that overlap each other in a depth direction at the designated position or a plurality of objects that include the designated position in a region of the object; and
receiving an input for an operation related to display for the target object.
